# EUROPÄISCHE PATENTSCHRIFT

(11) **EP 3 912 649 B1**
(45) Veröffentlichungstag und Bekanntmachung des Hinweises auf die Patenterteilung: **25.10.2023**
(21) Anmeldenummer: 21174485.9
(22) Anmeldetag: 18.05.2021
(51) Int. Cl.: A61L 9/20, A61L 2/00, A61L 2/10, A61L 2/24, F24F 8/22, E04H 1/12, F24F 120/10

(54) **MODULARER SCHUTZRAUM SOWIE DURCHREICHE FÜR EINEN SCHUTZRAUM**
MODULAR PROTECTIVE AREA AND PASS-THROUGH FOR SAME
ESPACE MODULAIRE DE PROTECTION, AINSI QUE PASSE-PAQUET POUR UN ESPACE DE PROTECTION

(30) Priorität: 19.05.2020 DE 102020113461; 19.05.2020 DE 202020102844 U
(43) Veröffentlichungstag der Anmeldung: 24.11.2021
(73) Patentinhaber: SEIWO Technik GmbH, 09430 Drebach, OT Scharfenstein (DE)
(72) Erfinder: Wabst, Jan, 09112 Chemnitz (DE); Köhler, Mike, 09456 Mildenau (DE); Arnold, Katrin, 09235 Burkhardtsdorf / OT Kemtau (DE)
(74) Vertreter: Rumrich, Gabriele

(56) Entgegenhaltungen:
- CH-A5- 609 126
- CN-Y- 2 629 705
- KR-B1- 101 674 482
- US-A1- 2004 261 324
- US-A1- 2005 211 415
- US-A1- 2015 025 300

## Beschreibung

Die vorliegende Erfindung betrifft einen modularen Schutzraum für eine oder mehrere Personen sowie eine Durchreiche für einen Schutzraum.

Vom Termin beim Notar über die Zulassung eines Fahrzeuges bin hin zur Unterschrift unter einem Kreditvertrag - im öffentlichen Zusammenleben gibt es zahlreiche Termine, die sich nicht mit einer Videokonferenz oder über ein Telefongespräch wahrnehmen lassen. Gerade im Hinblick auf mögliche auftretende Pandemien gewinnen Abstandsgebote und Hygienemaßnahmen immer mehr an Bedeutung. Ämter, öffentliche Verwaltungen, Dienstleister und viele Unternehmen mit häufigem Kundenkontakt stehen damit vor der Herausforderung, ihre Mitarbeiterinnen und Mitarbeiter sowie Kundinnen und Kunden bestmöglich vor einer potenziellen Ansteckung durch Viren zu schützen.

Einen vielversprechenden Ansatzpunkt bietet die räumliche Isolierung von Personen, die einen notwendigen Mindestabstand situationsbedingt nicht einhalten können.

Üblicherweise sind mobile Raumsysteme als Standardprofillösungen und in regelmäßiger kubischer Form ausgeprägt. Diese werden im Innenbereich als Besprechungsboxen oder im Außenbereich Container für Besprechungen, Aufenthalt oder Lagerung genutzt. Die herkömmlichen Raumsysteme sind nicht durch ein gesteuertes System von Gesundheitsschutzsystemen in Form von Lichtdesinfektion, Luftdesinfektion und antibakteriellen Oberflächen sowie kontaktloses Steuern der Funktionen im Anwenderbereich gekennzeichnet. Die herkömmlichen Raumsysteme verfügen im Regelfall maximal über eine Heizung oder Klimagerät sowie eine Stromversorgung für Licht und diverse Verbraucher. Die Eindämmung einer möglichen Viruslast erscheint somit schwierig.

Die Druckschrift DT 26 05 750 A1 beschreibt ein als bewegliche Isolierstation verwendetes und ausgerüstetes Großcontainergehäuse, aufweisend einen von einem Eingangsraum abgetrennten Isolierraum. Eine antivirale und/oder antibakterielle Behandlung des Innenraumes ist nicht vorgesehen, wodurch sich durch einen Benutzer eingebrachte Mikroorganismen unkontrolliert vermehren können.

In der Druckschrift DE 20 2005 008 334 U1 wird ein Reinraum, insbesondere für ein medizinisches oder biotechnologisches Labor beschrieben, welcher einen Innenraum und ein den Innenraum umfassendes Traggerüst aufweist. Paneele werden unter Einschluss von Dichtungselementen gegen Tragprofile des Traggerüstes gepresst, wodurch es zu einer hermetischen Abdichtung des Innenraums kommt. Auch hier ist eine antivirale und/oder antibakterielle Behandlung des Innenraumes nicht vorgesehen.

Die Druckschrift EP 1 588 720 A1 beschreibt eine zum mobilen Transport ausgebildete zur Desinfektion, Abtötung oder Passivierung pathogener Erreger an Organismen und/oder Gegenständen umfassend einen ersten Bereich mit zumindest einer Vorrichtung zum Versprühen einer Flüssigkeit, die ein oxidativ wirkendes Mittel enthält und einen zweiten Bereich mit zumindest einer UV-Lichtquelle.

Die Druckschrift EP 2 712 378 B1 beschreibt einen medizinischen Kiosk zur Fernberatung eines in einem Innenraum des medizinischen Kiosks befindlichen Benutzers, ausgestattet mit einem Videokonferenzsystem und mehreren medizinischen Fächern, die ferngesteuert verriegelbar sind. Weiterhin weist der medizinische Kiosk ein Reinigungs- oder Desinfektionssystem auf, welches ein UV-System beinhalten kann. Dieses weist mehrere UV-Lampen auf, die den Innenraum bei Abwesenheit eines Benutzers bestrahlen.

In der Druckschrift EP 3 087 227 B1 wird ein Gebäude, insbesondere ausgebildet als Krankenhaus, offenbart, umfassend eine Skelettstruktur aus mehreren Etagen, wobei einzelne Module mit einem oder mehreren Zimmern in die Skelettstruktur eingeschoben sind. Die Belieferung des Krankenhauses erfolgt über eine desinfizierende Schleuse, beispielsweise eine Schleuse, die UV-Lampen umfasst.

Aus den Druckschriften US 2015/025300 A1 und KR 101 674 482 B1 ist ebenfalls jeweils ein modularer Schutzraum mit einem Präsenzsensor und einer UV-Bestrahlungseinrichtung bekannt und die Dokumente US2005/211415A1 und US 2004/261324 A1 beschreiben jeweils einen modularen Schutzraum mit einer Luftbestrahlungsvorrichtung. Bei den vier vorgenannten Dokumenten ist es jedoch erforderlich, dass sich während der Bestrahlung keine Personen in den Räumen befinden.

Durchreichen mit einer hin- und her beweglichen Schiebemulde werden beispielsweise in den Druckschriften CH 609 126 A5 (Schiebemulde aus Panzerglas) und CN 2 629 705 Y (mit in der Schiebemulde angeordneten UV-Bestrahlungsvorrichtung) beschrieben.

Nachteilhafterweise sind die bekannten Lösungen entweder wenig flexibel und erlauben insbesondere nicht den Einsatz in Verbindung mit vorhandenen Räumlichkeiten, oder sie bieten keine Ausreichende Desinfektion des Innenraumes, bzw. nur unzureichenden Schutz vor der Ausbreitung von durch einen Benutzer eingetragenen Viren und Bakterien.

Der Erfindung liegt somit die Aufgabe zugrunde, eine flexibles, modulares, nachhaltig wiederverwertbares Raumsystem abzubilden, welches die Gesundheit der Nutzer und Anwender bestmöglich schützt.

Diese Aufgabe wird zum einen durch einen modularen Schutzraum für eine oder mehrere Personen gelöst, aufweisend
- wenigstens einen Innenraum, welcher durch wenigstens eine umfangsseitig geschlossene Wandung, einen Boden und eine Decke von wenigstens einem angrenzenden Raum abgeschlossen ist, wobei die umfangsseitig geschlossene Wandung durch wenigstens eine verschließbare Zugangsöffnung durchbrochen ist,
- wenigstens einen Präsenzsensor, welcher die Anwesenheit und/oder Abwesenheit einer Person in dem Innenraum detektiert, und
- wenigstens eine erste Bestrahlungsvorrichtung zur Bestrahlung des Innenraumes mit UVC-Strahlen.

Der Innenraum ist mindestens einseitig zugänglich und bildet einen allseitig geschlossenen Raum im Außenbereich und im Innenbereich einen mindestens fünfseitig geschlossenen Raum.

Vorteilhafterweise ist der erfindungsgemäße modulare Schutzraum in regelmäßiger kubischer Form ausgebildet. Alternativ können durch den erfindungsgemäßen modularen Schutzraum auch geschwungene bzw. organische Raumformen abgebildet werden.

Bevorzugt weist der erfindungsgemäße modulare Schutzraum wenigstens ein Luftreinigungssystem zur Filterung der in dem wenigstens einen Innenraum befindlichen Raumluft auf.

Dazu wird die Raumluft vorteilhafterweise durch ein Element zum Ansaugen der Abluft angesaugt, anschließend gefiltert und/oder bestrahlt und/oder auf sonstige weise gereinigt und schließlich über ein Element zum Abgeben der gereinigten Luft wieder in den Innenraum abgegeben.

Alternativ ist es möglich, die angesaugte Abluft entweder in gereinigter oder in ungereinigter Form in den angrenzenden Außenraum abzugeben und frische Luft aus dem Außenraum anzusaugen, die dann wahlweise gefiltert und/oder bestrahlt und/oder auf sonstige Weise gereinigt und schließlich über ein Element zum Abgeben der gereinigten Luft wieder in den Innenraum abgegeben werden kann.

Vorteilhafterweise ist der Präsenzsensor ein Bewegungsmelder, ein optischer Sensor, ein Wärmesensor, eine Kamera, oder ein kapazitiver Sensor oder sind wenigstens zwei der vorgenannten Sensoren miteinander kombiniert.

Der Präsenzsensor kann direkt an der Zugangsöffnung angebracht werden. Zwangsläufig wird der Präsenzsensor jedoch so angeordnet, dass er in der Lage ist zu erfassen, ob sich eine oder mehrere Personen in dem Innenraum des modularen Schutzraumes aufhalten.

Der erfindungsgemäße modulare Schutzraum weist weiterhin eine Steuerungseinheit auf, welche die vorhandenen Bestrahlungsvorrichtungen und/oder die Zugangsöffnung und/oder das Luftreinigungssystem und weitere elektronische Komponenten steuert und welche Signale von dem Präsenzsensor und von möglichen weiteren Sensoren, beispielsweise eines Temperatursensors, erfasst. Die Steuerung ist vorzugsweise eine intelligente Steuerung, wobei auch vorgegebene Routinen berücksichtigt werden können. Ebenfalls durch die Steuerungseinheit gesteuert werden kann beispielsweise ein Heizsystem.

Insbesondere bestrahlt die wenigstens eine erste Bestrahlungsvorrichtung bei einer durch den Präsenzsensor detektierten Anwesenheit wenigstens einer Person in dem Innenraum den Innenraum mit UVC-Strahlung mit einer Wellenlänge von 100 nm bis 230 nm, bevorzugt von 222 nm.

Eine Bestrahlung mit einer derartigen Wellenlänge kann vorteilhafterweise als permanente Hintergrundbestrahlung eingesetzt werden, da sie zwar bereits antiviral und antibakteriell wirkt, sich jedoch gleichzeitig nicht schädlich auf in dem Innenraum anwesende Personen auswirkt.

Im Innenraum befindliche Schattenbereiche, beispielsweise in der Region von Schränken, Tischen und dergleichen werden vorzugsweise mit weiteren UVC-Lampen ausgeleistet, um eine optimale Bestrahlung zu gewährleisten und ein Ansammeln von Krankheitserregern / Viren und/oder Bakterien zu vermeiden.

Gleichzeitig kann bei einem Betreten des Innenraumes durch eine oder mehrere Personen eine Innenraumbeleuchtung mit Licht im sichtbaren Wellenlängenbereich automatisch angeschaltet werden. Dieses Licht kann beispielsweise von Flächenlichter, Deckeneinbauspots und/oder Aufbaubeleuchtungseinheiten abgegeben werden.

Bevorzugt weist der erfindungsgemäße modulare Schutzraum wenigstens eine zweite Bestrahlungsvorrichtung auf, welche bei einer durch den Präsenzsensor detektierten Abwesenheit von Personen in dem Innenraum den Innenraum mit UVC-Strahlung mit einer Wellenlänge über 222 nm bestrahlt.

Vorzugsweise bestrahlt die zweite Bestrahlungsvorrichtung den Innenraum mit Licht einer Wellenlänge von 250 nm bis 290 nm.

Vorzugsweise weist der erfindungsgemäße modulare Schutzraum eine Zeitschaltuhr auf, welche die wenigstens eine zweite Bestrahlungsvorrichtung nach einem vorbestimmten Zeitintervall, bevorzugt nach ein bis vier Minuten, besonders bevorzugt nach 2 Minuten, abschaltet.

Die Zeitschaltuhr ist vorteilhafterweise mit der Steuerungseinrichtung gekoppelt.

Insbesondere weist der erfindungsgemäße modulare Schutzraum ein Zugangsverriegelungssystem auf, welches die Zugangsöffnung während der Dauer der Bestrahlung durch die wenigstens eine zweite Bestrahlungsvorrichtung versperrt.

Dadurch wird gewährleistet, dass keine Person mit der potentiell schädlichen UVC-Strahlung im höheren Wellenlängenbereich in Kontakt kommt.

Die Bestrahlungszeit durch die zweite Bestrahlungsvorrichtung kann durch eine Signallampe an der Außenseite des erfindungsgemäßen modularen Schutzraumes angezeigt werden.

Vorteilhafterweise ist in der wenigstens einen verschließbaren Zugangsöffnung eine bewegungsgesteuerte und/oder signalgesteuerte Automatiktür und/oder eine Tür mit mechanischer Verriegelung angeordnet.

Der Zugang erfolgt bevorzugt berührungslos, beispielsweise durch Stimmeingabe, Funksender, RFID-Sender oder sonstige berührungslose Öffnung bzw. Schließung.

Der Zugang ist zudem bevorzugt barrierefrei ausgestaltet.

Die Zugangsöffnung kann alternativ mit einer normgerechten manuell zu bedienender Tür ausgestattet werden.

Im Bereich der Zugangsöffnung kann zusätzlich eine weitere Desinfektionseinheit, beispielsweise zur Abgabe eines Handdesinfektionsmittels, angeordnet sein.

Bevorzugt sind in dem Innenraum befindliche Oberflächen aus einem antibakteriellen und/oder antivirulenten Material ausgebildet und/oder mit einem antibakteriellen und/oder antivirulenten Material beschichtet.

Dies betrifft insbesondere in Reichweite des Nutzers befindliche Flächen wie beispielsweise integrierte Tische oder Paneels.

Insbesondere ist ein antibakterielles und/oder antivirulentes Material ausgewählt aus Edelstahl, Aluminium Messing, Kunststoff, ionisiertem Kunststoff, Komposit-Materialien bestehend aus Kunststoff und mineralischen Bestandteilen, Holzwerkstoff mit einer Beschichtung aus antibakteriellen und/oder antivirulenten Material oder einer beliebigen Kombination der vorgenannten Materialien.

Bevorzugt weist das Luftreinigungssystem eine dritte Luftbestrahlungsvorrichtung auf, wobei die Raumluft durch das Luftreinigungssystem angesaugt wird, die angesaugte Raumluft durch die Luftbestrahlungsvorrichtung mit UVC-Strahlung mit einer Wellenlänge von 230 nm bis 280 nm, bevorzugt von 254 nm bis 265 nm bestrahlt wird und die bestrahlte Raumluft durch das Luftreinigungssystem wieder in den Innenraum abgegeben wird.

Alternativ oder zusätzlich sind auch andere Filtermethoden zur Filterung der angesaugten Abluft denkbar.

Alternativ kann durch das Luftreinigungssystem auch Außenluft angesaugt, bestrahlt und in den Innenraum abgegeben werden.

In einer bevorzugten Ausführungsform weist der erfindungsgemäße modulare Schutzraum wenigstens eine Innenwand zur Abtrennung eines ersten Innenraums von einem zweiten Innenraum oder zur Abtrennung des Innenraums von einem Bestandsgebäude auf.

Der erfindungsgemäße modulare Schutzraum kann beispielsweise auch an eine Außen- oder Innenwand eines Bestandsgebäudes angebaut werden.

Vorteilhafterweise kann sich in dem ersten Innenraum eine erste Person und in dem zweiten Innenraum eine zweite Peron aufhalten, die so in einem geschützten Umfeld miteinander kommunizieren können. Dazu ist wenigstens ein Teilbereich der Innenwand transparent und/oder transluzent ausgebildet.

Der modulare Schutzraum kann mit einer Wechselsprech- und/oder mit einer Audioanlage ausgestattet sein, so dass die Kommunikation zwischen beiden Kommunikatoren gewährleistet ist.

Vorteilhafterweise weist der erfindungsgemäße modulare Schutzraum wenigstens eine elektromechanische und/oder mechanische Durchreiche auf.

Die Durchreiche weist vorteilhafterweise eine Trennwand und zwei Öffnungsklappen auf, welche auf zwei verschiedenen Seiten der Trennwand angeordnet sind und welche den Zugang zu einer zwischen den Öffnungsklappen hin und her beweglichen Schiebemulde ermöglichen. Die Trennwand ist insbesondere transparent und/oder transluzent ausgebildet.

Vorzugsweise ist/sind in einem Innenraum der Schiebemulde wenigstens eine Flächenbestrahlungsvorrichtung und/oder wenigstens eine Muldenbestrahlungsvorrichtung zur Bestrahlung des Innenraumes der Schiebemulde mit UVC-Strahlen angeordnet.

Vorteilhafterweise kann die Schiebemulde elektromechanisch und/oder mechanisch bewegt werden. Dazu kann an einer oder an beiden Seiten der Durchreiche ein Betätigungshebel und/oder -schalter angeordnet sein.

Bevorzugt ist eine Bodenfläche der Schiebemulde aus UV-durchlässigem Glas oder UV-durchlässigem Kunststoff gebildet.

Ein Gegenstand oder Dokument, welches in die Schiebemulde gelegt wird, wird nach Schließen der Öffnungsklappen mit UVC-Strahlen bestrahlt und somit ebenfalls von Mikroorganismen gereinigt.

Bevorzugt bestrahlen die wenigstens eine Flächenbestrahlungsvorrichtung und/oder die wenigstens eine Muldenbestrahlungsvorrichtung den Innenraum der Schiebemulde bei geschlossenen Öffnungsklappen mit UVC-Strahlung mit einer Wellenlänge über 222 nm.

Die Steuerung der Schiebemulde inklusive ihrer Bestrahlungsvorrichtungen kann mit der Steuerungseinheit des erfindungsgemäßen modularen Schutzraumes gekoppelt sein.

Die Durchreiche kann in der Wandung des modularen Schutzraumes oder in einer Innenwand des modularen Schutzraumes angeordnet sein. Auch die Verwendung mehrerer Durchreichen in einem modularen Schutzraum ist denkbar.

Die Durchreiche kann auch ohne Desinfektionssystem oder mit einem alternativen Desinfektionssystem ausgestattet sein.

Bevorzugt weist der erfindungsgemäße modulare Schutzraum einen Temperatursensor zum Erfassen einer Körpertemperatur wenigstens einer in dem modularen Schutzraum befindlichen Person auf.

Bei einer erhöhten Körpertemperatur, die auf ein Infektionsrisiko hindeuten könnte, kann ein optisches und/oder akustisches Warnsignal abgegeben werden. Richtwerte für eine "reguläre" Körpertemperatur können in der Steuerungseinheit programmiert werden oder vorprogrammiert vorliegen.

Weiterhin bevorzugt weist der erfindungsgemäße modulare Schutzraum einen Bildschirm und/oder ein Videokonferenzsystem und/oder eine Kamera und/oder eine Scaneinheit und/oder einen Drucker und/oder eine Wechselsprechanlage und/oder eine Audioanlage und/oder eine oder mehrere Schnittstellen für einen Beamer, einen Laptop, einen Scanner und/oder einen Drucker auf.

Vorteilhafterweise weist der erfindungsgemäße modulare Schutzraum ein Heizsystem, insbesondere einen Doppelboden mit integrierter Infrarotheizung, auf.

Ein mit Doppelboden ausgestatteter modularer Schutzraum eignet sich insbesondere für die Anwendung im Außenbereich. Das Raumsystem für den Außenbereich besitzt bevorzugt eine regen-, wasser-, sturm-, schnee- und/oder sonnengesicherte wärmegedämmte Decke und kann zudem vandalismussicher ausgebildet werden.

Insbesondere sind auf einem umlaufenden Rahmenprofil mehrere senkrecht zu dem Rahmenprofil ausgerichtete Tragprofile angeordnet oder anbringbar, wobei an jeweils einem Tragprofil wenigstens zwei Einhangprofile angebracht oder anbringbar sind, wobei an jedem Einhangprofil wenigstens ein Füllelement angebracht oder anbringbar ist.

Das System für den Innenbereich ist vorzugsweise einwandig, kann jedoch auch doppelwandig ausgeführt werden. Es verfügt bevorzugt über eine Innenraumdecke mit der Möglichkeit Beleuchtungseinheiten zu integrieren (bspw. Flächenlichter, Deckeneinbauspots, Aufbaubeleuchtungseinheiten sowie das UVC-Desinfektionslicht oder Monitore bzw. Lauflichter, Projektoren).

Vorzugsweise ist ein Einhangprofil mittels wenigstens einer Aussparung in ein Formelement, welches in ein Tragprofil in Bezug auf das Tragprofil radial nach außen weisend angeordnet oder anbringbar ist, eingehangen oder einhängbar.

Alternativ oder zusätzlich kann eine Verbindung mittels Schrauben, Nieten, Schweißen oder ähnlichem erfolgen.

Durch das Einhängen wird die Montage und Demontage vereinfacht und es kann ein rascher, situationsbedingter Umbau des modularen Schutzraumes erfolgen.

Bevorzugt ist das wenigstens eine Füllelement aus einem Material ausgewählt aus Metall, Holzwerkstoffen, Kunststoffen, Verbundelementen, Mineralwerkstoffen, Acrylmineralwerkstoffen, transparenten oder transluszenten Werkstoffen, integrierten Bildschirmen und/oder aufprojizierenden Monitoren ausgewählt.

Insbesondere ist ein transparenter oder transluszenter Werkstoff Glas, schaltbares Glas und/oder Kunststoff.

Vorteilhafterweise ist das Glas Sicherheitsglas und besteht das Glas aus einer einlagigen Sicherheitsglasscheibe oder einer mehrlagigen Sicherheitsglas-Verbundscheibe.

Neben der einen oder mehreren Bestrahlungseinheit/en zur Bestrahlung mit UVC-Licht können weitere Bestrahlungseinheiten zur Beleuchtung des Innenraumes mit Licht im sichtbaren Wellenlängenbereich vorhanden sein.

An der Außenwandung kann in der Nähe der Zugangsöffnung ferner eine Signalleuchte angeordnet sein, die anzeigt, ob sich eine oder mehrere Personen in dem Innenraum aufhalten.

Die Aufgabe wird weiterhin gelöst durch einen Bausatz zur Herstellung eines erfindungsgemäßen modularen Schutzraumes, aufweisend wenigstens ein Rahmenprofil oder wenigstens zwei zu einem Rahmenprofil verbindbare Rahmenprofil-Elemente, wenigstens drei Tragprofile, wenigstens sechs Einhangprofile, wenigstens drei Füllelemente und wenigstens ein Deckenelement.

Bevorzugt weist der erfindungsgemäße Bausatz zur Herstellung eines erfindungsgemäßen modularen Schutzraumes wenigstens einen Präsenzsensor, welcher die Anwesenheit und/oder Abwesenheit einer Person in einem Innenraum des modularen Schutzraumes detektiert, und wenigstens eine erste Bestrahlungsvorrichtung zur Bestrahlung des Innenraumes mit UVC-Strahlen auf.

Insbesondere weist der erfindungsgemäße Bausatz zur Herstellung eines erfindungsgemä-ßen modularen Schutzraumes weiterhin ein Luftreinigungssystem zur Filterung der in dem wenigstens einen Innenraum befindlichen Raumluft auf.

Das Luftreinigungssystem beinhaltet vorzugsweise wenigstens ein Element zum Ansaugen der in dem Innenraum befindlichen Raumluft und ein Element zum Abgeben von Frischluft in den Innenraum. Weiterhin bevorzugt sind Elemente zur Bestrahlung der angesaugten Raumluft mit UVC-Strahlen und/oder Filterelemente enthalten.

Alle weiter oben im Rahmen der Beschreibung des erfindungsgemäßen modularen Schutzraumes beschriebenen Komponenten können auch in dem erfindungsgemäßen Bausatz zur Herstellung eines erfindungsgemäßen modularen Schutzraumes enthalten sein.

Die Aufgabe wird weiterhin gelöst durch einen Bausatz zur Umrüstung bestehender Räume in einen erfindungsgemäßen modularen Schutzraum, wobei der Bausatz mindestens ein UVC-Leuchtmittel und mindestens einen Präsenzsensor aufweist.

Vorzugsweise weist der erfindungsgemäße Bausatz zur Umrüstung bestehender Räume in einen erfindungsgemäßen modularen Schutzraum weiterhin eine Steuerungseinheit auf.

Die Steuerungseinheit ist vorteilhafterweise als intelligente Steuerung ausgebildet.

Alle weiter oben im Rahmen der Beschreibung des erfindungsgemäßen modularen Schutzraumes beschriebenen Komponenten können auch in dem erfindungsgemäßen Bausatz zur Umrüstung bestehender Räume in einen erfindungsgemäßen modularen Schutzraum enthalten sein.

Die Aufgabe wird ferner gelöst durch eine Durchreiche für einen Schutzraum aufweisend eine Trennwand und zwei Öffnungsklappen, welche auf zwei verschiedenen Seiten der Trennwand angeordnet sind und welche den Zugang zu einer zwischen den Öffnungsklappen hin und her beweglichen Schiebemulde ermöglichen, wobei erfindungsgemäß in einem Innenraum der Schiebemulde wenigstens eine Flächenbestrahlungsvorrichtung und/oder wenigstens eine Muldenbestrahlungsvorrichtung zur Bestrahlung des Innenraumes der Schiebemulde mit UVC-Strahlen angeordnet ist/sind.

Die Trennwand ist insbesondere transparent und/oder transluzent ausgebildet.

Vorteilhafterweise kann die Schiebemulde elektromechanisch und/oder mechanisch bewegt werden.

Dazu kann an einer oder an beiden Seiten der Durchreiche ein Betätigungshebel und/oder -schalter angeordnet sein.

Bevorzugt ist eine Bodenfläche der Schiebemulde aus UV-durchlässigem Glas oder UV-durchlässigem Kunststoff gebildet.

Bevorzugt bestrahlen die wenigstens eine Flächenbestrahlungsvorrichtung und/oder die wenigstens eine Muldenbestrahlungsvorrichtung den Innenraum der Schiebemulde bei geschlossenen Öffnungsklappen mit UVC-Strahlung mit einer Wellenlänge über 222 nm.

Der erfindungsgemäße modulare Schutzraum sowie die erfindungsgemäßen Bausätze und die erfindungsgemäße Durchreiche gewährleisten vorteilhafterweise eine nahezu bakterien- und virenfreien Kommunikation zwischen Personen im öffentlichen Bereich.

Aufgrund des Einsatzes von UVC-Licht werden bei einem entsprechend langen Einsatz Viren komplett vernichtet. Bei UVC-Licht in geringerer Dosis wird die Virenlast so geringgehalten, dass keine Gefahr mehr für die Gesundheit ausgeht. Hinzu kommt der Einsatz von antibakteriellen Oberflächen auf den Arbeitsbereichen und in Bereichen mit physischem Kontakt (Tisch- und Sitzflächen, Griffe). Diese Oberflächen minimieren das Wachstum von Mikroorganismen. Auf den Einsatz von Desinfektionsmitteln, die Flächen angreifen oder in höherer Konzentration die Gesundheit gefährden, kann verzichtet werden. Durch den Einsatz des modularen Schutzraumes, der Bausätze oder der Durchreiche kann auf das Tragen von Mund- und Nasenschutz in den Räumen verzichtet werden, was sich wiederum positiv auf die Gesprächssituation, die Arbeitsergebnisse und die Psyche aller Beteiligten auswirkt.

Vorteilhafterweise sind die erfindungsgemäßen Lösungen auf eine berührungslose Steuerung bzw. Nutzung ausgelegt.

Ein Einsatz der vorgeschlagenen Lösungen ist beispielsweise in Altenheimen, öffentlichen Behörden, Kanzleien und anderen Büroräumen mit häufigem Kundenkontakt, in Besprechungssituationen oder auf Messen denkbar. Die Systeme können ebenfalls im Eingangsbereich von Großveranstaltungen wie Konzerten, Messen, Fußballspielen oder Aufsichtsratssitzungen Anwendung finden.

Die Erfindung wird nachfolgend anhand von Ausführungsbeispielen und zugehörigen Figuren näher erläutert, ohne dabei auf diese beschränkt zu sein.

Es zeigen:
- Figur 1: eine schematische Darstellung eines Innenraums eines erfindungsgemäßen modularen Schutzraumes;
- Figur 2: einen Grundriss einer Ausführungsform eines erfindungsgemäßen modularen Schutzraumes mit einem Innenraum;
- Figur 3: einen Grundriss einer Ausführungsform eines erfindungsgemäßen modularen Schutzraumes mit zwei Innenräumen;
- Figur 4: einen Grundriss einer weiteren Ausführungsform eines erfindungsgemäßen modularen Schutzraumes;
- Figur 5: einen Grundriss einer weiteren Ausführungsform eines erfindungsgemäßen modularen Schutzraumes mit Videokonferenzsystem;
- Figur 6: einen Grundriss einer weiteren Ausführungsform eines erfindungsgemäßen modularen Schutzraumes mit mehreren Zugängen;
- Figur 7: eine schematische Ansicht einer Durchreiche eines erfindungsgemäßen modularen Schutzraumes;
- Figur 8: einen Längsschnitt durch eine Durchreiche eines erfindungsgemäßen modularen Schutzraumes;
- Figur 9: eine Explosionsdarstellung des Verbindungsbereiches zwischen Tragprofil und Rahmenprofil eines erfindungsgemäßen modularen Schutzraumes;
- Figur 10: einen Querschnitt durch einen Wandbereich eines erfindungsgemäßen modularen Schutzraumes;
- Figur 11: eine schematische Ansicht des Verbindungsbereiches zwischen Tragprofil und Einhangprofil eines erfindungsgemäßen modularen Schutzraumes;
- Figur 12: eine Schnittdarstellung eines Wandbereiches eines für die Innenbereichsnutzung vorgesehenen erfindungsgemäßen modularen Schutzraumes;
- Figur 13: eine Schnittdarstellung eines Wandbereiches eines für die Außenbereichsnutzung vorgesehenen erfindungsgemäßen modularen Schutzraumes mit sichtbarem Tragprofil; und
- Figur 14: eine Schnittdarstellung eines Wandbereiches eines weiteren für die Außenbereichsnutzung vorgesehenen erfindungsgemäßen modularen Schutzraumes mit unsichtbarem Tragprofil.

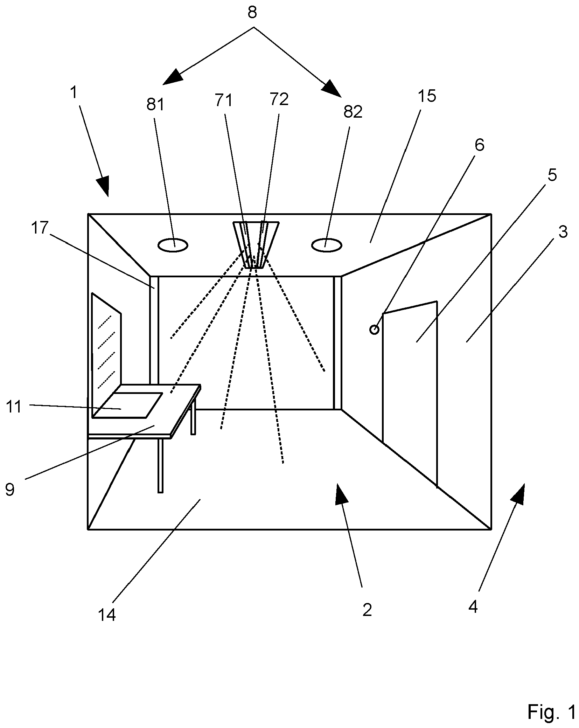

In **Figur 1** ist eine schematische Darstellung eines Innenraums 2 eines erfindungsgemäßen modularen Schutzraumes 1 gezeigt. Der Innenraum 2 ist umgeben von einer umfangsseitig geschlossenen Wandung 3, welche lediglich durch eine Zugangsöffnung 5 durchbrochen wird und den Innenraum 2 von einem angrenzenden Raum 4 abtrennt. Der modulare Schutzraum 1 weist weiterhin einen Boden 14 und eine Decke 15 auf, wobei in der Decke 15 mehrere Einlässe für Bestrahlungsvorrichtungen 71, 72 und ein Luftreinigungssystem 8 angeordnet sind.

Eine erste Bestrahlungsvorrichtung 71 ist in der dargestellten Ausführungsform als UV-LED-Lampe ausgebildet und gibt bei Anwesenheit mindestens einer Person in dem Innenraum 2 UVC-Strahlung mit einer Wellenlänge von etwa 222 nm in den Innenraum 2 ab. Die Anwesenheit einer Person wird dabei mittels eines Präsenzsensors 6 festgestellt, der beispielsweise ein Bewegungsmelder sein kann. Im vorliegenden Ausführungsbeispiel ist der Präsenzsensor 6 in der Wandung 3 angeordnet; er kann jedoch ebenfalls in der Decke 15 oder an einer sonstigen Position befindlich sein, von welcher aus er den Innenraum 2 erfassen kann.

Eine zweite Bestrahlungsvorrichtung 72 ist in der dargestellten Ausführungsform ebenfalls als UV-LED-Lampe ausgebildet und gibt bei Abwesenheit einer Person in dem Innenraum 2 UVC-Strahlung mit einer Wellenlänge von 250 nm bis 290 nm in den Innenraum 2 ab. Die Abwesenheit einer Person wird ebenfalls durch den Präsenzsensor 6 festgestellt. Nach Verlassen des Raums ist der Raum für mindestens 2 Minuten elektronisch gesperrt und das UVC Licht (mit einer Frequenz zwischen 250 und 290 Nanometern) wird zugeschaltet und desinfiziert den Raum und die Oberflächen von Bakterien und Viren.

Die Leistungsaufnahme der zweiten Bestrahlungsvorrichtung 72 beträgt zwischen 10 und 25W und ist für Räume bis 15qm möglich. Größere Räume bedürfen ergänzenden UVC Lichtquellen. Es kann pro 15 qm Innenraum 2 ein Leuchtmittel als Leuchtstoffröhre oder ein voll- oder teilflächiges LED UVC Licht eingesetzt werden. Zusätzliche Möbel oder Einbauten werden mit zusätzlichen UVC Lichtquellen beschienen, um etwaige Schattenwürfe auszugleichen. Jede Kubatur des Raumes sowie etwaige Inneneinbauten werden berücksichtigt.

In der Zugangsöffnung 5 ist eine vorzugsweise automatische Tür vorgesehen, welche über einen weiteren nicht dargestellten Bewegungsmelder geöffnet werden kann. Vorzugsweise weist der Schutzraum ein ebenfalls nicht dargestelltes Zugangsverriegelungssystem auf, welches die Tür bei Bestrahlung des Innenraumes 2 durch die zweite Bestrahlungsvorrichtung 72 verriegelt, sodass ein ungewolltes Betreten durch einen Benutzer verhindert wird. Über eine ebenfalls nicht dargestellte Zeitschaltuhr kann die Dauer der Bestrahlung durch die zweite Bestrahlungsvorrichtung 72 sowie der Zugangsverriegelung geregelt werden.

In der Decke 15 können optional weitere nicht dargestellte Beleuchtungsvorrichtungen zur Bestrahlung des Innenraumes 2 mit sichtbarem Licht vorgesehen sein.

Das Luftreinigungssystem 8 weist in der dargestellten Ausführungsform ein Element zum Ansaugen der Abluft 81 und ein Element zum Ausströmen der Zuluft 82 auf. Die angesaugte Abluft wird mittels einer nicht dargestellten dritten Bestrahlungsvorrichtung mit UVC-Strahlung mit einer Wellenlänge von 254 nm bis 265 nm bestrahlt. Alternativ oder zusätzlich sind auch andere Filtermethoden zur Filterung der angesaugten Abluft denkbar. Die Leistungsaufnahme der dritten Bestrahlungsvorrichtung beträgt in einer bevorzugten Ausführungsform 20 - 135 W; 55 - 350m³/ h. Die Geräte können der Raumgröße angepasst werden.

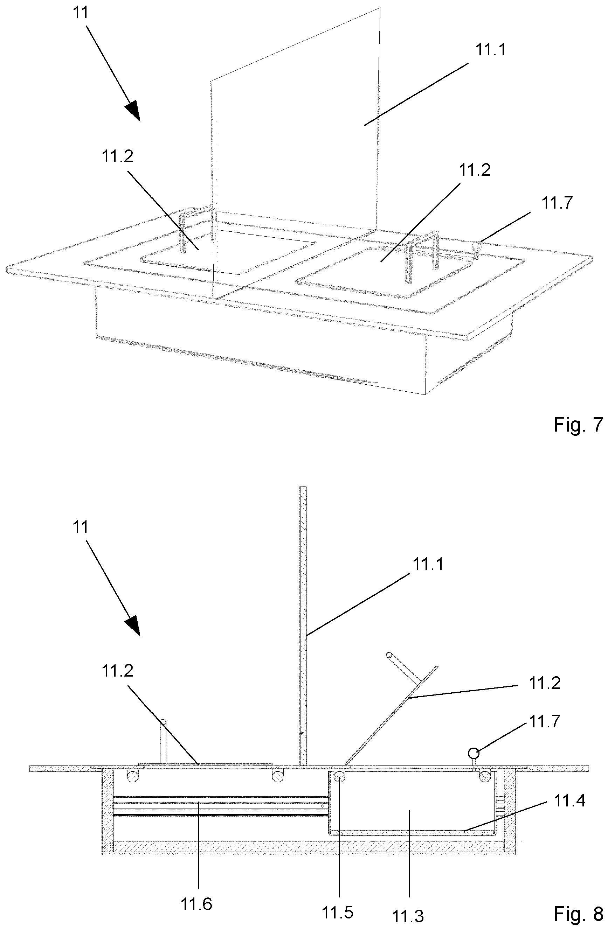

In dem Innenraum 2 ist im dargestellten Ausführungsbeispiel weiterhin eine Tischplatte vorhanden, deren Oberfläche 9 mit einem antivirulenten Material beschichtet ist. Weitere nicht dargestellte Oberflächen wie Stühle und/oder technische Geräte können ebenfalls vorhanden sein und sind bevorzugt ebenfalls mit einem antibakteriellen und/oder antivirulenten Material beschichtet oder aus einem solchen Material gefertigt. In die Tischplatte integriert ist eine Durchreiche 11, deren genaue Funktionsweise in den Figuren 7 und 8 erläutert wird.

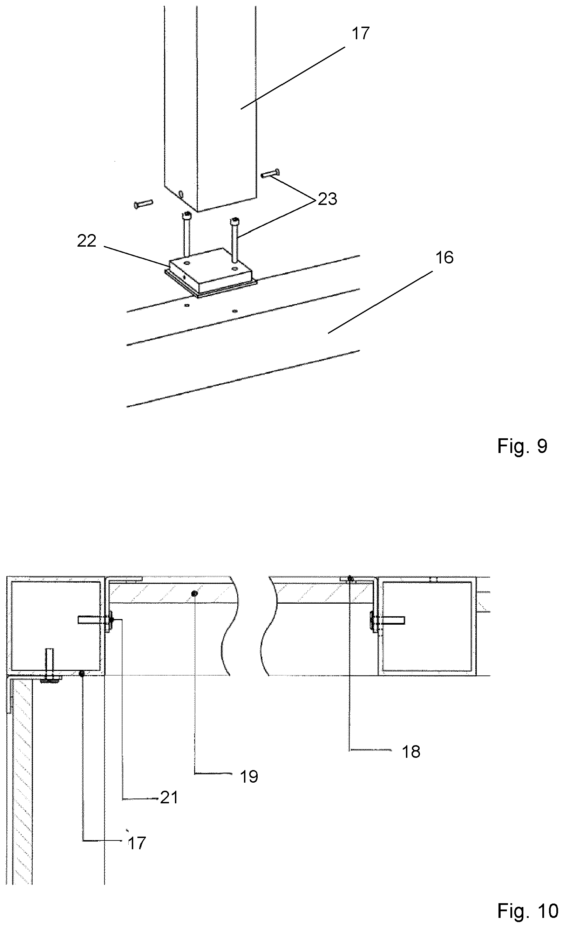

Weiterhin erkennbar sind Tragprofile 17, deren Zusammenwirkung mit weiteren Elementen in den Figuren 9 bis 14 erläutert wird.

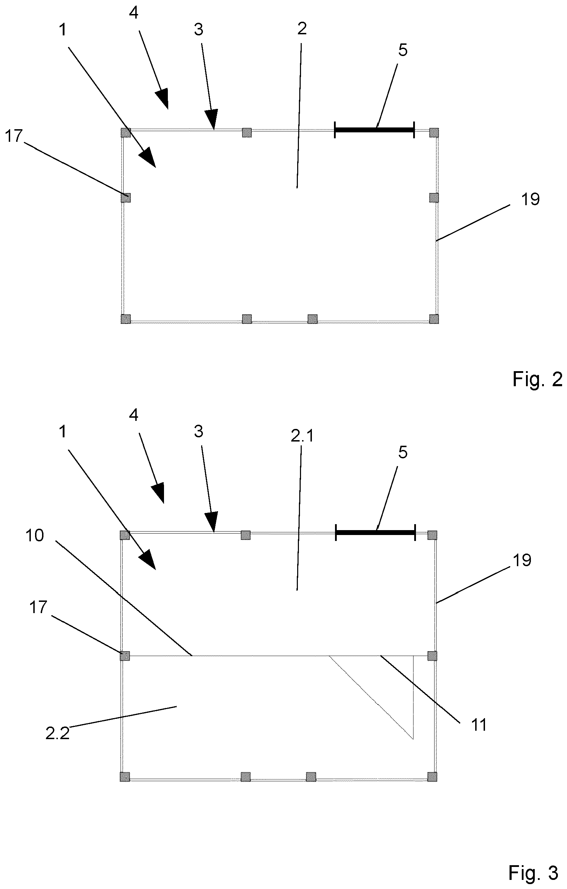

Die **Figuren 2 bis 6** zeigen Grundrisse von verschiedenen Ausführungsformen des erfindungsgemäßen modularen Schutzraumes 1. Die umfangsseitig geschlossene Wandung 3 wird dabei stets durch Tragprofile 17, Füllelemente 19 und nicht dargestellte Einhangprofile gebildet. Die Tragprofile 17 sind in einem festgelegten Raster auf einem Rahmenprofil angeordnet. In allen Ausführungsformen der Figuren 2 bis 6 wird die Wandung 3 durch zumindest eine Zugangsöffnung 5 durchbrochen in welcher bevorzugt eine verriegelbare, automatisch gesteuerte Tür angeordnet ist.

Ein Einraumsystem als digitaler Medienraum dient zum Abwickeln von Behördenvorgängen mit einem direkten oder medial mittels Softwarealgorhythmus geführtem Kommunikationssystem durch eine audiovisuelle Schnittstelle (bspw. Bildschirm mit Kamera und Audioanlage, Projektor mit Kamera- oder Bewegungssensorik und Audioausgabe/ Audioeingabe), zusätzlich kann der Einraum mit weiterer Technik für Drucken, Kopieren, Dokumentenaustausch (bspw. Durchreichen oder Briefkästen), Audioeingabe, Gestensteuerung ergänzt werden. Der Raum ist mindestens fünf-seitig verschlossen mit mindestens einem Zugang innerhalb von Gebäuden und außerhalb von Gebäuden als Außenraum-Solitärvariante mindestens sechsseitig geschlossen mit mindestens einem Zugang. Grundsätzlich ist der Einraum mit mindestens einer passiven Desinfektionseinheit mittels UVC Licht, doppelstufigem UVC Licht oder/ und Luftdesinfektion versehen. Weiterhin kann das Einraumsystem mit einer mechanischen oder elektronischen, steuerbaren oder nichtsteuerbaren Schließanlage ausgestattet werden. Ein elektronisches Zugangskontrollsystem ermöglich Termin- und Belegungsplanung. Der Zugang erfolgt über eine Sicherheitstür über eine Klinkenbedienung mit antibakterieller Klinke (vorzugsweise Edelstahl) oder über eine automatische Tür mittels elektronischem Zugang, mittels Codeeingabe oder berührungslos. Das Raumsystem kann einzeln in Bestandteilen oder als Gesamtheit vormontiert transportiert werden. Die Maße können variieren, ebenso kann es sich beim Raum um einen Quader-, Würfel- oder ovalen oder Rundraum bzw. anderweitig geformten Raum handeln.

In der in **Figur 3** dargestellten Ausführungsform wird durch eine Innenwand 10 ein erster Innenraum 2.1 von einem zweiten Innenraum 2.2 abgetrennt. Zwischen dem ersten Innenraum 2.1 und dem zweiten Innenraum 2.2 ist eine Durchreiche 11 angeordnet. Ein Besucher kann durch die Zugangsöffnung 5 den ersten Innenraum 2.1 betreten und vorzugsweise durch eine Sicherheitsglasscheibe und Gegensprechanlage mit einer in dem zweiten Innenraum 2.2 befindlichen Person, beispielsweise einem Mitarbeiter oder einem Berater, kommunizieren. Durch die Durchreiche 11 können Dokumente oder kleinere Gegenstände ausgetauscht werden. Der Zutritt in den zweiten Innenraum 2.2 kann über die Wandung 3 oder die Innenwand 10 erfolgen.

Grundsätzlich wird auch bei der Ausführung als Zweiraum- oder Doppelraumsystem im Innenraum mit antibakteriellen Oberflächen oder normalen Oberflächenbeschichtungen gearbeitet. Der Raum ist allseitig geschlossen und verfügt mittig oder versetzt über eine physische Raumtrennung. Die Raumtrennung kann teilweise oder ganz verglast oder mit anderen Materialien geschlossen sein. Das Doppelraumsystem ist mit einer Wechselsprechanlage oder einem ähnlich funktionierenden Audiosystem ausgestattet und dient der Kommunikation ohne einen physischen Kontakt aufzubauen. Zudem ist das System beid- oder einseitig mit einer oder mehrere Desinfektionssystemen (wie UVC-, doppeltem- oder mehrfachstufigem UVC-Licht 222nm und/ 250 bis 280 nm UVC Licht, Luftdesinfektion) ausgestattet. Damit bietet der Raum ein Minimum an Ansteckungsgefahr. Der Zugang erfolgt jeweils getrennt über eine Tür, die auch vorzugsweise als elektronisch gesteuerte Automatiktür betrieben werden kann. Ein Raum dient als Kundenraum und der gegenüberliegende als Administrationsraum. Der Administrator kann sämtliche Funktionen im Kundenraum manuellen steuern. Beide Räume können mit Bürokommunikationssystemen, wie Drucker, Kopierer, Scanner ausgestattet werden. Zudem kann zwischen den Räumen eine Durchreiche für den Austausch von Dokumenten und/ oder Waren mit und ohne Desinfektionseinheit (bspw. durch UVC Licht) installiert sein.

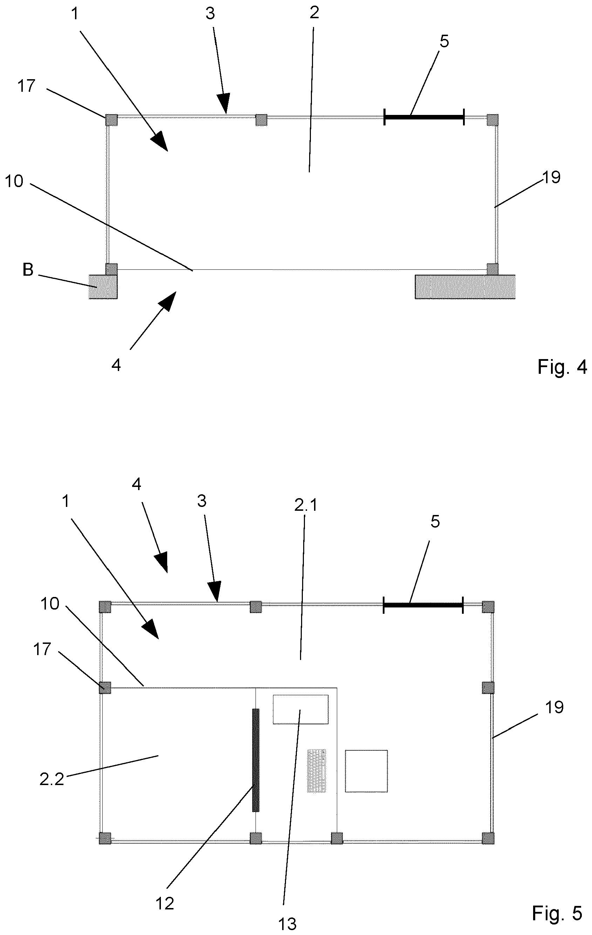

Die **Figur 4** zeigt eine Ausführungsform eines modularen Schutzraumes 1, welcher an eine Wand eines Bestandsgebäudes B angeordnet wird. Dabei kann es sich um eine Außenwand oder eine Innenwand eines Bestandsgebäudes B handeln. Eine Abtrennung des Innenraumes 2 von einem Außenraum 4 erfolgt hier beispielsweise dadurch, dass der Schutzraum 1 an eine Außenfassade eines Gebäudes B angesetzt wird, wobei eine sichere Abdichtung zum Gebäude B z.B. durch entsprechende Dichtungsmaterialien wie Silikon, Quellband und dergleichen erfolgt.

Eine im Außenraum 4 befindliche Person kann dabei mit einer im Innenraum 2 befindlichen Person ebenfalls durch eine Sicherheitsglasscheibe und Gegensprechanlage kommunizieren. Auch eine (nicht dargestellte) Durchreiche kann vorhanden sein.

Die in **Figur 5** dargestellte Ausführungsform zeigt ebenfalls einen modularen Schutzraum, bei dem durch eine Innenwand 10 ein erster Innenraum 2.1 von einem zweiten Innenraum 2.2 abgetrennt ist. Der zweite Innenraum 2.2 kann beispielsweise als Technikraum verwendet werden. In dem ersten Innenraum 2.1 befinden sich unter anderem ein Videokonferenzsystem 12 mit Bildschirm und Kamera, sowie weitere Geräte, wie beispielsweise Drucker 13, Scanner und/oder Eingabegeräte. Die Maße eines solchen modularen Schutzraumes können beispielhaft mit 4m Länge, 3m Breite und 2,5m Höhe angegeben werden.

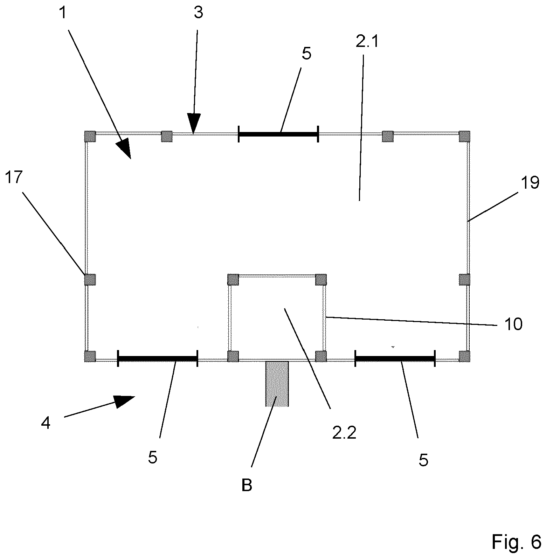

In **Figur 6** ist eine Ausführungsform gezeigt, die beispielsweise bei einem beschränkten Außenbauraum im Innenraum eines Gebäudes bzw. eines Raumes eines Gebäudes angeordnet ist. Durch eine Innenwand 10 ist auch hier ein erster Innenraum 2.1 von einem zweiten Innenraum 2.2 abgetrennt, der als Technikraum genutzt werden kann. Der modulare Schutzraum 1 weist drei Zugangsöffnungen 5 auf, von denen beispielsweise einer als Eingang und zwei als Ausgang oder zwei als Eingang und einer als Ausgang genutzt werden könnten um einen kontrollierten Besucherstrom in ein Bestandsgebäude B hinein oder aus diesem heraus zu führen. Es könnten zudem Durchreichen beispielsweise zu außerhalb liegenden Kassenhäuschen vorhanden sein.

Die **Figur 7** zeigt eine schematische Ansicht einer Durchreiche 11 eines erfindungsgemä-ßen modularen Schutzraumes. Die Durchreiche 11 kann dabei zwischen einem ersten Innenraum und einem zweiten Innenraum oder zwischen einem Innenraum und einem Außenraum des modularen Schutzraumes angeordnet sein. Sie weist eine Trennscheibe 11.1 auf, durch welche sich zwei Personen, welche die Durchreiche 11 benutzen, sehen können. Vorteilhafterweise ist auch eine nicht dargestellte Gegensprechanlage vorhanden. Auf jeder Seite der Trennscheibe 11.1 befindet sich eine Öffnungsklappe 11.2, die den Zugang zu einer darunter liegenden Schiebemulde erlaubt. Auf einer Seite der Trennscheibe 11.1 ist ein Betätigungshebel 11.7 angeordnet, durch welchen die Schiebemulde auf die gegenüberliegende Seite und zurück bewegt werden kann. Es kann zusätzlich ein zweiter Betätigungshebel auf der gegenüberliegenden Seite vorgesehen sein. Alle Oberflächen der Durchreiche sind mit einem antibakteriellen und/oder antiviralen Material beschichtet oder aus einem solchen Material gebildet.

In dem in **Figur 8** dargestellten Längsschnitt durch eine Durchreiche 11 eines erfindungsgemäßen modularen Schutzraumes wird die Funktionsweise der Durchreiche 11 verdeutlicht. Nach dem Öffnen einer Öffnungsklappe 11.2 kann ein Dokument oder kleinerer Gegenstand in die Schiebemulde 11.3 eingelegt werden. Die Öffnungsmulde wird anschließend geschlissen und eine Flächenbestrahlungsvorrichtung 11.4 bestrahlt den in der Mulde befindlichen Gegenstand / das Dokument von der Unterseite mit UVC-Strahlen mit einer Wellenlänge 254 nm, während mehrere Muldenbestrahlungsvorrichtungen 11.5 den in der Mulde befindlichen Gegenstand / das Dokument von oben mit UVC-Strahlen bestrahlen. Der Boden der Schiebemulde 11.3 ist vorteilhafterweise aus UV-durchlässigem Material, vorzugsweise UV-durchlässigem Glas gebildet. Der Benutzer kann die Schiebemulde 11.3 mit dem Betätigungshebel 11.7 auf die gegenüberliegende Seite der Trennscheibe 11.1 schieben, wobei die Schiebemulde 11.3 an Führungsschienen 11.6 entlang geführt wird. Ein zweiter Benutzer kann die Öffnungsklappe 11.2 auf seiner Seite der Trennscheibe 11.1 nun öffnen und die desinfizierten Gegenstände / Dokumente entnehmen.

Die Fachgröße der Schiebemulde 11.3 kann variabel ausgebildet sein. Alternativ zum hier dargestellten mechanischen Bewegen durch einen Betätigungshebel 11.7 kann die Bewegung der Schiebemulde 11.3 elektrisch erfolgen.

Die Flächenbestrahlungsvorrichtung 11.4 und die Muldenbestrahlungsvorrichtungen 11.5 sind so ausgebildet, dass eine Bestrahlung des Innenraumes der Schiebermulde 11.3 nur bei geschlossenen Öffnungsklappen 11.2 erfolgt.

Die **Figur 9** zeigt eine Explosionsdarstellung des Verbindungsbereiches zwischen Tragprofil 17 und Rahmenprofil 16 eines erfindungsgemäßen modularen Schutzraumes. Über Schrauben 23 wird ein Adapterelement 22 an dem Rahmenprofil 16 befestigt. Auf das Adapterelement 22 wird ein Tragprofil 17 aufgesetzt und ebenfalls mittels Schrauben 23 befestigt. Die Medien (Netzwerk- und Stromkabel) sind im Profil oder außerhalb in geschützten Kanälen verlaufend und steckerfertig (mit Zwischenkupplung) vorinstalliert.

In **Figur 10** ist ein Querschnitt durch einen Wandbereich eines erfindungsgemäßen modularen Schutzraumes dargestellt. An einem Tragprofil 17 wird mittels eines Formelementes 21 ein Einhangprofil 18 befestigt. Dabei können an einem Tragprofil 17 mehrere Einhangprofile 18 angeordnet sein. Jedes Einhangprofil 18 ist mit einem Füllmaterial 19 verbunden, wobei die Verbindung zwischen Einhangprofil 18 und Füllmaterial 19 je nach Material durch Verkleben, Verschweißen, Vernieten oder ähnliche Befestigungsmethoden erfolgen kann.

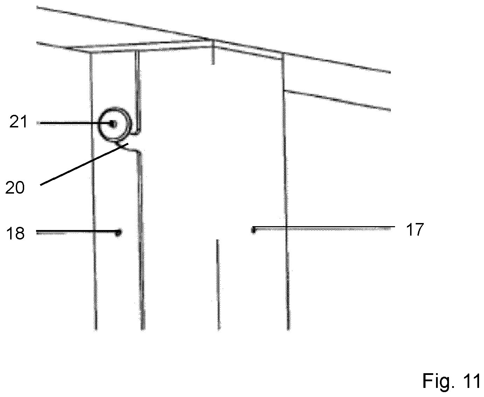

Die **Figur 11** zeigt eine schematische Ansicht des Verbindungsbereiches zwischen Tragprofil 17 und Einhangprofil 18 eines erfindungsgemäßen modularen Schutzraumes. In dem Tragprofil 17 ist ein in Bezug auf das Tragprofil 17 radial nach außen weisendes Formelement 21, beispielsweise eine Schraube, angeordnet. Das Formelement 21 kann jedoch auch ein Stift sein. Das zugehörige Einhangprofil 18 weist eine entsprechende zungenförmige Aussparung 20 auf, über welche das Einhangprofil auf das Formelement 21 eingehangen werden kann. Durch ein Festziehen der Schraube (Formelement 21) erfolgt eine Aussteifung der Verbindung. Die Konstruktion ist vorteilhafterweise einfach montierbar und demontierbar.

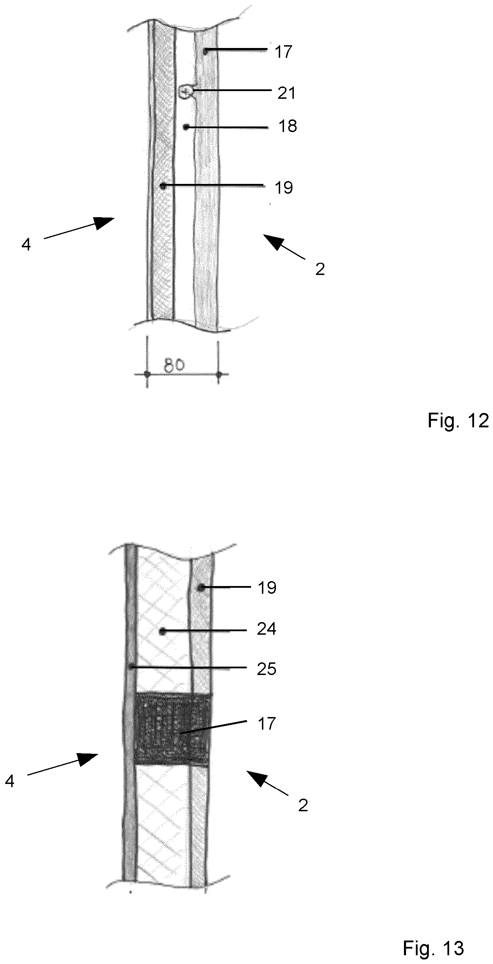

**Figur 12** zeigt eine Schnittdarstellung eines Wandbereiches eines für die Innenbereichsnutzung vorgesehenen erfindungsgemäßen modularen Schutzraumes. Der Wandbereich grenzt einen Außenbereich 4 von dem Innenraum 2 des modularen Schutzraumes ab. Das Tragprofil 17 ist ein 80 cm x 80 cm Aluprofil. Über ein Formelement 21 ist ein Einhangprofil 18 eingehängt, an welchem ein Füllmaterial 19 angebracht ist.

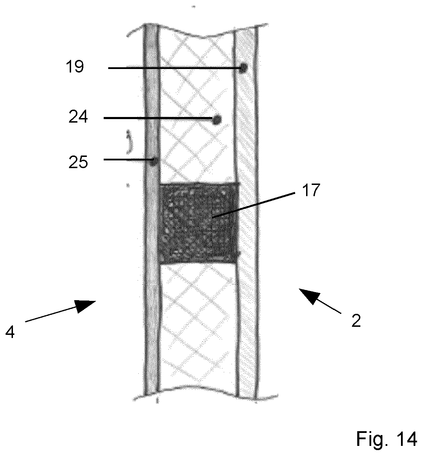

Die **Figur 13** zeigt eine Schnittdarstellung eines Wandbereiches eines für die Außenbereichsnutzung vorgesehenen erfindungsgemäßen modularen Schutzraumes mit sichtbarem Tragprofil 17, die **Figur 14** eine Schnittdarstellung eines Wandbereiches eines weiteren für die Außenbereichsnutzung vorgesehenen erfindungsgemäßen modularen Schutzraumes mit unsichtbarem Tragprofil 17.

Bei der in Figur 13 dargestellten Ausführungsform ist das Füllmaterial 19 derart am Tragprofil 17 angebracht (über ein nicht dargestelltes Einhangprofil), dass das Tragprofil vom Innenraum 2 aus sichtbar ist. Zum Außenbereich 4 hin ist eine Außenverkleidung 25 angebracht. Zwischen Außenverkleidung 25 und Füllmaterial 19 ist eine Wärmedämmung 24 angeordnet.

Bei der in Figur 14 dargestellten Ausführungsform ist das Füllmaterial 19 derart am Tragprofil 17 angebracht (über ein nicht dargestelltes Einhangprofil), dass das Tragprofil vom Innenraum 2 aus nicht sichtbar ist. Zum Außenbereich 4 hin ist eine Außenverkleidung 25 angebracht. Zwischen Außenverkleidung 25 und Füllmaterial 19 ist eine Wärmedämmung 24 angeordnet.

In beiden Varianten ist das Tragprofil 17 aus Holz mit den Maßen 80 cm x 100 cm ausgebildet.

### Bezugszeichenliste

- 1: modularer Schutzraum
- 2: Innenraum
- 2.1: erster Innenraum
- 2.2: zweiter Innenraum
- 3: Wandung
- 4: angrenzender Raum
- 5: Zugangsöffnung
- 6: Präsenzsensor
- 71: erste Bestrahlungsvorrichtung
- 72: zweite Bestrahlungsvorrichtung
- 8: Luftreinigungssystem
- 81: Abluft
- 82: Zuluft
- 9: Oberfläche
- 10: Innenwand
- 11: Durchreiche
- 11.1: Trennscheibe
- 11.2: Öffnungsklappe
- 11.3: Schiebemulde
- 11.4: Flächenbestrahlungsvorrichtung
- 11.5: Muldenbestrahlungsvorrichtung
- 11.6: Führungsschiene
- 11.7: Betätigungshebel
- 12: Videokonferenzsystem
- 13: Drucker
- 14: Boden
- 15: Decke
- 16: Rahmenprofil
- 17: Tragprofil
- 18: Einhangprofil
- 19: Füllelement
- 20: Aussparung
- 21: Formelement
- 22: Adapterelement
- 23: Schrauben
- 24: Wärmedämmung
- 25: Außenverkleidung
- B: Bestandsgebäude

## Patentansprüche

1. Modularer Schutzraum (1) für eine oder mehrere Personen, aufweisend
• wenigstens einen Innenraum (2), welcher durch wenigstens eine umfangsseitig geschlossene Wandung (3), einen Boden (14) und eine Decke (15) von wenigstens einem angrenzenden Außenraum (4) abgeschlossen ist, wobei die umfangsseitig geschlossene Wandung (3) durch wenigstens eine verschließbare Zugangsöffnung (5) durchbrochen ist,
• wenigstens einen Präsenzsensor (6), welcher die Anwesenheit und/oder Abwesenheit einer Person in dem Innenraum (2) detektiert, und
• wenigstens eine erste Bestrahlungsvorrichtung (71) zur Bestrahlung des Innenraumes (2) mit UVC-Strahlen, wobei die wenigstens eine erste Bestrahlungsvorrichtung (71) dazu ausgelegt ist, bei einer durch den Präsenzsensor (6) detektierten Anwesenheit wenigstens einer Person in dem Innenraum (2) den Innenraum mit UVC-Strahlung mit einer Wellenlänge von 100 nm bis 230 nm zu bestrahlen,
• wenigstens eine zweite Bestrahlungsvorrichtung (72), derart ausgelegt, dass die zweite Bestrahlungsvorrichtung (72) bei einer durch den Präsenzsensor detektierten Abwesenheit von Personen in dem Innenraum (2) den Innenraum (2) mit UVC-Strahlung mit einer Wellenlänge über 222 nm bestrahlt,
- ein Zugangsverriegelungssystem, dazu ausgelegt, während der Dauer der Bestrahlung durch die wenigstens eine zweite Bestrahlungsvorrichtung (72) die Zugangsöffnung (5) zu versperren,
• wenigstens ein Luftreinigungssystem (8) zur Filterung der in dem wenigstens einen Innenraum (2) befindlichen Raumluft, wobei das Luftreinigungssystem (8) eine dritte Luftbestrahlungsvorrichtung aufweist und derart ausgelegt ist, dass die Raumluft
durch das Luftreinigungssystem (8) angesaugt wird, die angesaugte Raumluft durch die Luftbestrahlungsvorrichtung mit UVC-Strahlung mit einer Wellenlänge von 230 nm bis 280 nm bestrahlt wird und die bestrahlte Raumluft durch das Luftreinigungssystem (8) wieder in den Innenraum abgegeben wird,
• in dem Innenraum befindliche Oberflächen (9), die aus einem antibakteriellen und/oder antivirulenten Material ausgebildet und/oder mit einem antibakteriellen und/oder antivirulenten Material beschichtet sind.

2. Modularer Schutzraum (1) nach Anspruch 1, **dadurch gekennzeichnet, dass** der Präsenzsensor (6) ein Bewegungsmelder, ein optischer Sensor, ein Wärmesensor, eine Kamera oder ein kapazitiver Sensor ist oder dass wenigstens zwei der vorgenannten Sensoren miteinander kombiniert sind.

3. Modularer Schutzraum (1) nach einem der Ansprüche 1 oder 2, **dadurch gekennzeichnet, dass** die wenigstens eine erste Bestrahlungsvorrichtung (71) dazu ausgelegt ist, bei einer durch den Präsenzsensor (6) detektierten Anwesenheit wenigstens einer Person den Innenraum mit UVC-Strahlung mit einer Wellenlänge von 222 nm zu bestrahlen.

4. Modularer Schutzraum (1) nach Anspruch 1 oder 2, aufweisend eine Zeitschaltuhr, dazu ausgelegt, die wenigstens eine zweite Bestrahlungsvorrichtung (72) nach einem vorbestimmten Zeitintervall, bevorzugt nach ein bis vier Minuten, besonders bevorzugt nach 2 Minuten, abzuschalten.

5. Modularer Schutzraum (1) nach einem der Ansprüche 1 bis 3, **dadurch gekennzeichnet, dass** in der wenigstens einen verschließbaren Zugangsöffnung (5) eine bewegungsgesteuerte und/oder signalgesteuerte Automatiktür und/oder eine Tür mit mechanischer Verriegelung angeordnet ist.

6. Modularer Schutzraum (1) nach Anspruch 5, **dadurch gekennzeichnet, dass** ein antibakterielles und/oder antivirulentes Material ausgewählt ist aus Edelstahl, Aluminium Messing, Kunststoff, ionisiertem Kunststoff, Komposit-Materialien bestehend aus Kunststoff und mineralischen Bestandteilen, Holzwerkstoff mit einer Beschichtung aus antibakteriellen und/oder antivirulenten Material oder einer beliebigen Kombination der vorgenannten Materialien.

7. Modularer Schutzraum (1) nach Anspruch 1, **dadurch gekennzeichnet, dass** das Luftreinigungssystem (8) dazu ausgelegt ist, die angesaugte Raumluft durch die Luftbestrahlungsvorrichtung mit UVC-Strahlung mit einer Wellenlänge von 254 nm bis 265 nm zu bestrahlen.

8. Modularer Schutzraum (1) nach einem der Ansprüche 1 bis 7, aufweisend wenigstens eine elektromechanische und/oder mechanische Durchreiche (11).

9. Modularer Schutzraum (1) nach einem der Ansprüche 1 bis 8, aufweisend einen Temperatursensor zum Erfassen einer Körpertemperatur wenigstens einer in dem modularen Schutzraum befindlichen Person.

10. Modularer Schutzraum (1) nach einem der Ansprüche 1 bis 9, weiterhin aufweisend einen Bildschirm und/oder ein Videokonferenzsystem (12) und/oder eine Kamera und/oder eine Scaneinheit und/oder einen Drucker (13) und/oder eine Wechselsprechanlage und/oder eine Audioanlage und/oder eine oder mehrere Schnittstellen für einen Beamer, einen Laptop, einen Scanner und/oder einen Drucker.

11. Modularer Schutzraum (1) nach einem der Ansprüche 1 bis 10 aufweisend ein Heizsystem, insbesondere einen Doppelboden mit integrierter Infrarotheizung.

12. Modularer Schutzraum (1) nach einem der Ansprüche 1 bis 11, **dadurch gekennzeichnet, dass** auf einem umlaufenden Rahmenprofil (16) mehrere senkrecht zu dem Rahmenprofil ausgerichtete Tragprofile (17) angeordnet sind und, dass an jeweils einem Tragprofil (17) wenigstens zwei Einhangprofile (18) angebracht sind, wobei an jedem Einhangprofil (18) wenigstens ein Füllelement (19) ist.

13. Modularer Schutzraum (1) nach Anspruch 12, **dadurch gekennzeichnet, dass** ein Einhangprofil (18) mittels wenigstens einer Aussparung (20) in ein Formelement (21), welches in ein Tragprofil (17) im Bezug auf das Tragprofil (17) radial nach außen weisend angeordnet ist, eingehangen ist.

14. Modularer Schutzraum (1) nach Anspruch 12 oder 13, **dadurch gekennzeichnet, dass** das wenigstens eine Füllelement (19) aus einem Material ausgewählt aus Metall, Holzwerkstoffen, Kunststoffen, Verbundelementen, Mineralwerkstoffen, Acrylmineralwerkstoffen, transparenten oder transluszenten Werkstoffen, integrierten Bildschirmen und/oder aufprojizierenden Monitoren ausgewählt ist.

15. Modularer Schutzraum (1) nach Anspruch 14, **dadurch gekennzeichnet, dass** ein transparenter oder transluszenter Werkstoff Glas, schaltbares Glas und/oder Kunststoff ist.

16. Modularer Schutzraum (1) nach Anspruch 15, **dadurch gekennzeichnet, dass** das Glas Sicherheitsglas ist und aus einer einlagigen Sicherheitsglasscheibe oder einer mehrlagigen Sicherheitsglas-Verbundscheibe besteht.

17. Durchreiche (11) für einen Schutzraum, aufweisend eine Trennwand (11.1) und zwei Öffnungsklappen (11.2), welche auf zwei verschiedenen Seiten der Trennwand (11.1) angeordnet sind und welche den Zugang zu einer zwischen den Öffnungsklappen (11.2) hin und her beweglichen Schiebemulde (11.3) ermöglichen, **dadurch gekennzeichnet, dass** in einem Innenraum der Schiebemulde (11.3) wenigstens eine Flächenbestrahlungsvorrichtung (11.4) und/oder wenigstens eine Muldenbestrahlungsvorrichtung (11.5) zur Bestrahlung des Innenraumes der Schiebemulde (11.3) mit UVC-Strahlen angeordnet ist und dass eine Bodenfläche der Schiebemulde (11.3) aus UV-durchlässigem Glas oder UV-durchlässigem Kunststoff gebildet ist.

18. Durchreiche (11) nach Anspruch 17, **dadurch gekennzeichnet, dass** die Schiebemulde (11.3) elektromechanisch und/oder mechanisch bewegt werden kann.

19. Durchreiche nach einem der Ansprüche 17 oder 18, **dadurch gekennzeichnet, dass** die wenigstens eine Flächenbestrahlungsvorrichtung (11.4) und/oder die wenigstens eine Muldenbestrahlungsvorrichtung (11.5) dazu ausgelegt ist, den Innenraum der Schiebemulde (11.3) bei geschlossenen Öffnungsklappen (11.2) mit UVC-Strahlung mit einer Wellenlänge über 222 nm zu bestrahlen.

## Claims

1. Modular shelter (1) for one or more persons, comprising
• at least one interior space (2), which is closed off from at least one adjoining exterior space (4) by at least one peripherally closed wall (3), a floor (14) and a ceiling (15), wherein the peripherally closed wall (3) is pierced by at least one closable access opening (5),
• at least one presence sensor (6), which detects the presence and/or absence of a person in the interior space (2), and
• at least one first irradiation device (71) for irradiating the interior space (2) with UVC radiation, wherein the at least one first irradiation device (71) is designed to irradiate the interior space with UVC radiation having a wavelength of 100 nm to 230 nm when the presence of at least one person in the interior space (2) is detected by the presence sensor (6),
• at least one second irradiation device (72), designed in such a way that the second irradiation device (72) irradiates the interior space (2) with UVC radiation having a wavelength above 222 nm in the event of an absence of persons in the interior space (2) detected by the presence sensor,
- an access interlock system adapted to block the access opening (5) for the duration of irradiation by the at least one second irradiation device (72),
• at least one air purification system (8) for filtering the room air located in the at least one interior space (2), wherein the air purification system (8) has a third air irradiation device and is designed in such a way that the room air is drawn in by the air purification system (8), the drawn-in room air is irradiated by the air irradiation device with UVC radiation having a wavelength of 230 nm to 280 nm, and the irradiated room air is discharged again into the interior space by the air purification system (8),
• surfaces (9) located in the interior space, which are formed from an antibacterial and/or antivirulent material and/or are coated with an antibacterial and/or antivirulent material.

2. Modular shelter (1) according to claim 1, **characterized in that** the presence sensor (6) is a motion sensor, an optical sensor, a thermal sensor, a camera or a capacitive sensor, or **in that** at least two of the aforementioned sensors are combined with each other.

3. Modular shelter (1) according to one of claims 1 or 2, **characterized in that** the at least one first irradiation device (71) is adapted to irradiate the interior space with UVC radiation having a wavelength of 222 nm upon a presence of at least one person detected by the presence sensor (6).

4. Modular shelter (1) according to claim 1 or 2, comprising a timer, adapted to switch off the at least one second irradiation device (72) after a predetermined time interval, preferably after one to four minutes, particularly preferably after 2 minutes.

5. Modular shelter (1) according to one of claims 1 to 3, **characterized in that** a motioncontrolled and/or signal-controlled automatic door and/or a door with mechanical locking is arranged in the at least one lockable access opening (5).

6. Modular shelter (1) according to claim 5, **characterized in that** an antibacterial and/or antivirulent material is selected from stainless steel, aluminum brass, plastic, ionized plastic, composite materials consisting of plastic and mineral components, wood-based material with a coating of antibacterial and/or antivirulent material, or any combination of the aforementioned materials.

7. Modular shelter (1) according to claim 1, **characterized in that** the air purification system (8) is adapted to irradiate the drawn-in room air by the air irradiation device with UVC radiation having a wavelength of 254 nm to 265 nm.

8. Modular shelter (1) according to one of claims 1 to 7, comprising at least one electromechanical and/or mechanical pass-through (11).

9. Modular shelter (1) according to one of claims 1 to 8, comprising a temperature sensor for detecting a body temperature of at least one person located in the modular shelter.

10. Modular shelter (1) according to one of claims 1 to 9, further comprising a screen and/or a video conferencing system (12) and/or a camera and/or a scanning unit and/or a printer (13) and/or an intercom system and/or an audio system and/or one or more interfaces for a beamer, a laptop, a scanner and/or a printer.

11. Modular shelter (1) according to one of claims 1 to 10, comprising a heating system, in particular a raised floor with integrated infrared heating.

12. Modular shelter (1) according to one of claims 1 to 11, **characterized in that** a plurality of support profiles (17) aligned perpendicularly to the frame profile are arranged on a circumferential frame profile (16), and **in that** at least two hook-in profiles (18) are attached to a respective support profile (17), wherein at least one filler element (19) is on each hook-in profile (18).

13. Modular shelter (1) according to claim 12, **characterized in that** a hook-in profile (18) is suspended by means of at least one recess (20) in a shaped element (21) arranged in a support profile (17) facing radially outwards with respect to the support profile (17).

14. Modular shelter (1) according to claim 12 or 13, **characterized in that** the at least one filler element (19) is selected from a material selected from metal, wood materials, plastics, composite elements, mineral materials, acrylic mineral materials, transparent or translucent materials, integrated screens and/or front-projecting monitors.

15. Modular shelter (1) according to claim 14, **characterized in that** a transparent or translucent material is glass, switchable glass and/or plastic.

16. Modular shelter (1) according to claim 15, **characterized in that** the glass is safety glass and consists of a single-layer safety glass pane or a multilayer safety glass laminated pane.

17. Pass-through (11) for a shelter, comprising a partition wall (11.1) and two opening flaps (11.2) which are arranged on two different sides of the partition wall (11.1) and which allow access to a sliding tray (11.3) movable to and fro between the opening flaps (11.2), **characterized in that** in an interior space of the sliding tray (11.3) at least one surface irradiation device (11.4) and/or at least one tray irradiation device (11.5) for irradiating the interior space of the sliding tray (11.3) with UVC rays is arranged, and **in that** a bottom surface of the sliding tray (11.3) is formed from UV-permeable glass or UV-permeable plastic.

18. Pass-through (11) according to claim 17, **characterized in that** the sliding tray (11.3) can be moved electromechanically and/or mechanically.

19. Pass-through according to one of claims 17 or 18, **characterized in that** the at least one surface irradiation device (11.4) and/or the at least one tray irradiation device (11.5) is designed to irradiate the interior space of the sliding tray (11.3) with UVC radiation having a wavelength above 222 nm when the opening flaps (11.2) are closed.

## Revendications

1. Espace modulaire de protection (1) pour une ou plusieurs personnes, présentant
• au moins un espace intérieur (2) qui est fermé par au moins une paroi (3) fermée sur son pourtour, un fond (14) et un plafond (15) par rapport à au moins un espace extérieur (4) adjacent, la paroi (3) fermée sur son pourtour étant percée d'au moins une ouverture d'accès (5) fermable,
• au moins un capteur de présence (6) qui détecte la présence et/ou l'absence d'une personne dans l'espace intérieur (2), et
• au moins un premier dispositif d'irradiation (71) pour irradier l'espace intérieur (2) avec des rayons UVC, l'au moins un premier dispositif d'irradiation (71) étant conçu pour irradier l'espace intérieur avec un rayonnement UVC d'une longueur d'onde de 100 nm à 230 nm en cas de présence d'au moins une personne dans l'espace intérieur (2) détectée par le capteur de présence (6),
• au moins un deuxième dispositif d'irradiation (72), conçu de telle sorte que le deuxième dispositif d'irradiation (72), en cas d'absence de personnes dans l'espace intérieur (2) détectée par le capteur de présence, irradie l'espace intérieur (2) avec un rayonnement UVC d'une longueur d'onde supérieure à 222 nm,
- un système de verrouillage d'accès conçu pour bloquer l'ouverture d'accès (5) pendant la durée de l'irradiation par l'au moins un deuxième dispositif d'irradiation (72),
• au moins un système de purification d'air (8) pour filtrer l'air ambiant se trouvant dans l'au moins un espace intérieur (2), le système de purification d'air (8) présentant un troisième dispositif d'irradiation d'air et étant conçu de telle sorte que l'air ambiant est aspiré par le système de purification d'air (8), l'air ambiant aspiré est irradié par le dispositif d'irradiation d'air avec un rayonnement UVC d'une longueur d'onde de 230 nm à 280 nm et l'air ambiant irradié est à nouveau libéré dans l'espace intérieur par le système de purification d'air (8),
• des surfaces (9) situées dans l'espace intérieur, qui sont formées d'un matériau antibactérien et/ou antivirulent et/ou sont revêtues d'un matériau antibactérien et/ou antivirulent.

2. Espace modulaire de protection (1) selon la revendication 1, **caractérisé en ce que** le capteur de présence (6) est un détecteur de mouvement, un capteur optique, un capteur thermique, une caméra ou un capteur capacitif, ou **en ce qu'**au moins deux des capteurs susmentionnés sont combinés entre eux.

3. Espace modulaire de protection (1) selon l'une des revendications 1 ou 2, **caractérisé en ce que** l'au moins un premier dispositif d'irradiation (71) est conçu pour, en cas de présence d'au moins une personne détectée par le capteur de présence (6), irradier l'espace intérieur avec un rayonnement UVC d'une longueur d'onde de 222 nm.

4. Espace modulaire de protection (1) selon la revendication 1 ou 2, présentant une minuterie conçue pour arrêter l'au moins un deuxième dispositif d'irradiation (72) après un intervalle de temps prédéterminé, de préférence après une à quatre minutes, plus préférablement après 2 minutes.

5. Espace modulaire de protection (1) selon l'une des revendications 1 à 3, **caractérisé en ce que** dans l'au moins une ouverture d'accès (5) fermable est disposée une porte automatique commandée par mouvement et/ou par signal et/ou une porte avec verrouillage mécanique.

6. Espace modulaire de protection (1) selon la revendication 5, **caractérisé en ce qu'**un matériau antibactérien et/ou antiviral est choisi parmi l'acier inoxydable, le laiton d'aluminium, le plastique, le plastique ionisé, les matériaux composites constitués de plastique et de composants minéraux, le matériau en bois avec un revêtement en matériau antibactérien et/ou antivirulent ou une combinaison quelconque des matériaux précités.

7. Espace modulaire de protection (1) selon la revendication 1, **caractérisé en ce que** le système de purification d'air (8) est conçu pour irradier l'air ambiant aspiré par le dispositif d'irradiation d'air avec un rayonnement UVC d'une longueur d'onde de 254 nm à 265 nm

8. Espace modulaire de protection (1) selon l'une des revendications 1 à 7, présentant au moins un passe-paquet (11) électromécanique et/ou mécanique.

9. Espace modulaire de protection (1) selon l'une des revendications 1 à 8, présentant un capteur de température pour détecter une température corporelle d'au moins une personne se trouvant dans l'espace modulaire.

10. Espace modulaire de protection (1) selon l'une des revendications 1 à 9, présentant en outre un écran et/ou un système de vidéoconférence (12) et/ou une caméra et/ou une unité de balayage et/ou une imprimante (13) et/ou un interphone et/ou un système audio et/ou une ou plusieurs interfaces pour un vidéoprojecteur, un ordinateur portable, un scanner et/ou une imprimante.

11. Espace modulaire de protection (1) selon l'une des revendications 1 à 10, présentant un système de chauffage, en particulier un faux plancher avec chauffage infrarouge intégré.

12. Espace modulaire de protection (1) selon l'une des revendications 1 à 11, **caractérisé en ce que** plusieurs profilés de support (17) orientés perpendiculairement au profilé de cadre sont disposés sur un profilé de cadre périphérique (16) et **en ce qu'**au moins deux profilés d'accrochage (18) sont montés sur un profilé de support respectif (17), au moins un élément de remplissage (19) étant présent sur chaque profilé d'accrochage (18).

13. Espace modulaire de protection (1) selon la revendication 12, **caractérisé en ce qu'**un profilé d'accrochage (18) est accroché au moyen d'au moins un évidement (20) dans un élément de forme (21) qui est disposé dans un profilé de support (17) en étant orienté radialement vers l'extérieur par rapport au profilé de support (17).

14. Espace modulaire de protection (1) selon la revendication 12 ou 13, **caractérisé en ce que** l'au moins un élément de remplissage (19) est choisi dans un matériau choisi parmi le métal, les matériaux en bois, les matières plastiques, les éléments composites, les matériaux minéraux, les matériaux minéraux acryliques, les matériaux transparents ou translucides, les écrans intégrés et/ou les moniteurs à projection.

15. Espace modulaire de protection (1) selon la revendication 14, **caractérisé en ce qu'**un matériau transparent ou translucide est du verre, du verre commutable et/ou du plastique.

16. Espace modulaire de protection (1) selon la revendication 15, **caractérisé en ce que** le verre est du verre de sécurité et est constitué d'une vitre en verre de sécurité à une couche ou d'une vitre en verre de sécurité feuilleté à plusieurs couches.

17. Passe-paquet (11) pour un espace de protection, présentant une paroi de séparation (11.1) et deux volets d'ouverture (11.2) qui sont disposés sur deux côtés différents de la paroi de séparation (11.1) et qui permettent l'accès à une cuvette coulissante (11.3) mobile en va-et-vient entre les volets d'ouverture (11.2), **caractérisé en ce que** dans un espace intérieur de la cuvette coulissante (11.3) est disposé au moins un dispositif d'irradiation de surface (11.4) et/ou au moins un dispositif d'irradiation de cuvette (11.5) pour irradier l'espace intérieur de la cuvette coulissante (11.3) avec des rayons UVC et **en ce qu'**une surface de fond de la cuvette coulissante (11.3) est formée de verre perméable aux UV ou de plastique perméable aux UV.

18. Passe-paquet (11) selon la revendication 17, **caractérisé en ce que** la cuvette coulissante (11.3) peut être déplacée de manière électromécanique et/ou mécanique.

19. Passe-paquet selon l'une des revendications 17 ou 18, **caractérisé en ce que** l'au moins un dispositif d'irradiation de surface (11.4) et/ou l'au moins un dispositif d'irradiation de cuvette (11.5) est conçu pour irradier l'espace intérieur de la cuvette coulissante (11.3) avec un rayonnement UVC d'une longueur d'onde supérieure à 222 nm lorsque les volets d'ouverture (11.2) sont fermés.
